# EUROPEAN PATENT APPLICATION

(11) **EP 2 157 164 A1**
(43) Date of publication of application: **24.02.2010**
(21) Application number: 08751796.7
(22) Date of filing: 22.05.2008
(51) Int. Cl.: C12M 1/34, G01N 21/27, G01N 33/48, G01N 33/483

(54) **OBSERVATION DEVICE**

(30) Priority: 24.05.2007 JP 2007137490
(71) Applicant: Nikon Corporation, Chiyoda-ku Tokyo 100-8331 (JP)
(72) Inventor: KIYOTA, Yasujiro, Tokyo 100-8331 (JP)
(74) Representative: Zeitler - Volpert - Kandlbinder
(86) International application number: PCT/JP2008/001284
(87) International publication number: WO 2008/146474

(57) **Abstract**

An observation device which is provided with an observation optical system and observes a specimen cultured in a culture vessel via the observation optical system includes a moving unit moving the culture vessel to a position where a distortion caused by the observation optical system occurs, an imaging unit imaging the culture vessel to generate an image at the position where the distortion occurs, and a calculating unit calculating a medium volume of a medium in the culture vessel based on the image.

## Description

### TECHNICAL FIELD

The present invention relates to an observation device to observe a specimen which is cultured in a culture vessel.

### BACKGROUND ART

In an observation device to observe a specimen which is cultured in a culture vessel, such a technique has been contemplated conventionally that culture condition of the specimen is determined based on an image which is generated through imaging the specimen (refer to the Patent Document 1 , for example). In the invention according to the Patent Document 1, culture condition is determined based on the image through measurement of the size of cell area or number of cells, which is the object to culture.
Patent Document 1: Japanese Unexamined Patent Application Publication No. 2004-16194

### DISCLOSURE OF THE INVENTION

### PROBLEMS TO BE SOLVED BY THE INVENTION

There are, however, various elements in culture condition, not only aforementioned size of cell area or number of cells. For example, information related to culture environment such as variation of medium volume and variation of medium pH is also important. The medium volume may happen to change by evaporation of medium and the like, whereby it is vitally important to grasp the variation of medium volume and the cause thereof.

A proposition of the present invention is to provide an observation device that can grasp the culture condition in detail based on the medium volume in the culture vessel, without adding any new arrangement thereto.

### MEANS FOR SOLVING THE PROBLEMS

An observation device according to the present invention, which is provided with an observation optical system and observes a specimen cultured in a culture vessel via the observation optical system includes a moving unit which moves the culture vessel to a position where a distortion caused by the observation optical system occurs, an imaging unit which images the culture vessel to generate an image at the position where the distortion occurs, and a calculating unit which calculates a medium volume of a medium in the culture vessel based on the image.

Note that it is preferable that the calculating unit extracts an edge of a bottom face of the culture vessel and an edge of a face of the medium, and may calculate the medium volume based on the edges which are extracted.

It is preferable that the observation device further includes an optical unit which can be put into and taken out from a light path of the observation optical system, and enhances the distortion.

An observation device according to the present invention which has an observation optical system and a stage on which a culture vessel being formed with one or a plurality of wells is placed, and observes, via the observation optical system, cells in a medium which is filled in each of the wells, it includes an imaging unit which has a plurality of imaging elements and images the culture vessel via the observation optical system to generate an image, an image processing unit which processes the image for each of the wells of the culture vessel based on the image generated by the imaging unit to obtain either an edge of a medium liquid level and an edge of a top face of the well or the edge of the medium liquid level and an edge of a bottom face of the well, and a calculating unit which calculates information related to a medium volume of the well based on the edge of the medium liquid level and the edge of the top face of the well or the edge of the bottom face of the well which are obtained by the image processing unit.

Note that it is preferable that the image processing unit includes a first image processing unit which performs edge enhancement process on the image for each of the wells of the culture vessel based on the image generated by the imaging unit and distinguishes to obtain the edge of the top face of the well, the edge of the medium liquid level, and the edge of the bottom face of the well, and a second image processing unit which calculates liquid level height information which is a distance between both edges viewed in radial direction from an optical axis of the observation optical system based on the edge of the medium liquid level and the edge of the bottom face of the well which are obtained by the first image processing unit, and calculates well depth information which is a distance between the both edges viewed in radial direction from the optical axis of the observation optical system based on the edge of the bottom face of the well and the edge of the top face of the well which are obtained by the first image processing unit, and in which the calculating unit calculates the information related to the medium volume based on the well depth information and the liquid level height information when it is determined that the liquid level height information has been obtained as information of two or more image elements of the imaging unit.

It is preferable that the observation device further includes a condition detecting unit which detects health condition of both the medium and the cells in the well based on an image of an area where an image at inner side of the edge of the top face of the well and an image at inner side of the edge of the bottom face of the well overlap, the edges being obtained by the first image processing unit.

It is further preferable that the stage and the observation optical system are moved relatively for the calculating unit to calculate the medium volume of all of the wells of the culture vessel, and the imaging unit images the culture vessel.

### BRIEF DESCRIPTION OF THE DRAWINGS

Fig. 1 is a block diagram illustrating constitution of a microscope according to an embodiment of the present invention.
Fig. 2 shows an example of a culture vessel which is an object of observation for the microscope 1 according to an embodiment of the present invention.
Fig. 3 is a flow chart illustrating an operation of computer control unit 31 when medium volume is calculated.
Fig. 4 is a depiction illustrating an example of an image when distortion occurs.

### BEST MODE FOR CARRYING OUT THE INVENTION

Hereinafter, an embodiment of the present invention will be described referring to the drawings. Note that, in the embodiment, a microscope is used for description as an example of an observation device of the present invention.

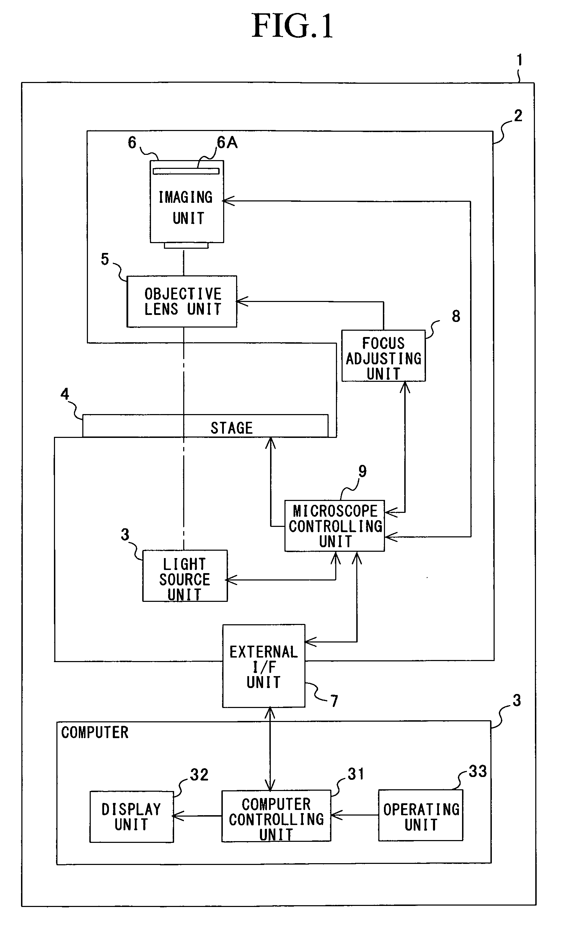

Fig. 1 is a block diagram illustrating constitution of a microscope according to an embodiment of the present invention. As shown in Fig. 1, the microscope 1 is constituted of a microscope body 2 and a computer 3.

The microscope body 2 is provided with a light source unit 3 including a lighting components such as white LED, a condensing lens, an aperture, and the like; a stage 4 on which a specimen of observation object is placed at a stand still; an objective lens 5; and imaging unit 6. In addition, the microscope body 2 is provided with an external I/F unit 7 which can be coupled with a computer 3 with each other, a focus controlling unit 8 which controls focus, a microscope controlling unit 9 which controls each unit. The microscope controlling unit 9 is coupled with the light source unit 3, the imaging unit 6, the external I/F unit 7, the focus controlling unit 8 with each other, and controls the stage 4.

The objective lens unit 5 is provided with an objective lens, an objective lens driving unit, an intermediate magnifying converter, and the like. Note that such a constitution may be applied that a plurality of objective lenses are provided to be changed over. The objective lens unit 5 has so-called bird-view optical system including a wide-angle lens (which has, for example, focus depth is not less than 40 mm, and a visual field of equal to or more than 80° horizontally x 60° vertically). The imaging unit 6 is provided with an imaging element 6A and an image processing unit which carries out image processing such as A/D conversion.

It should be noted that the microscope 1 may include members for fluorescence observation and members for phase difference observation, and the like, in addition to the constitution shown in Fig. 1.

The computer 3 is provided with a computer controlling unit 31, a displaying unit 32, an operation unit 33 so as to accept users' instruction regarding with the operation of the microscope 1 via the operation unit 33, and to display images obtained by the microscope 1 on the display unit 32. The computer controlling unit 31 stores programs in advance to control each unit in a memory, which is not shown. Then, the computer controlling unit 31 is coupled with the microscope controlling unit 9 via the external I/F unit 7 so as to obtain images generated by the imaging unit 6 from the microscope controlling unit 9 and to control the microscope controlling unit 9.

In observation by the microscope 1, the light source unit 3 irradiates illuminating light toward the culture vessel 40 placed at stand still on the stage 4. Illuminating light illuminates the culture vessel 40 and the specimen in the culture vessel 40. The light flux passed through the specimen is lead to the imaging unit 6 after penetrating the objective lens unit 5. The imaging unit 6 generates an image through imaging the penetrated image through the culture vessel 40 and the specimen via the imaging element 6A.

The microscope controlling unit 9 performs, by moving the stage 4 vertically, focus adjustment through altering the face which is the object for focus adjustment and moving a part of the objective lens unit 5 in the direction of an optical axis of the objective lens via the focus adjustment unit 8. Note that the focus adjustment is performed in a similar manner with a known technique, whereby the description is omitted.

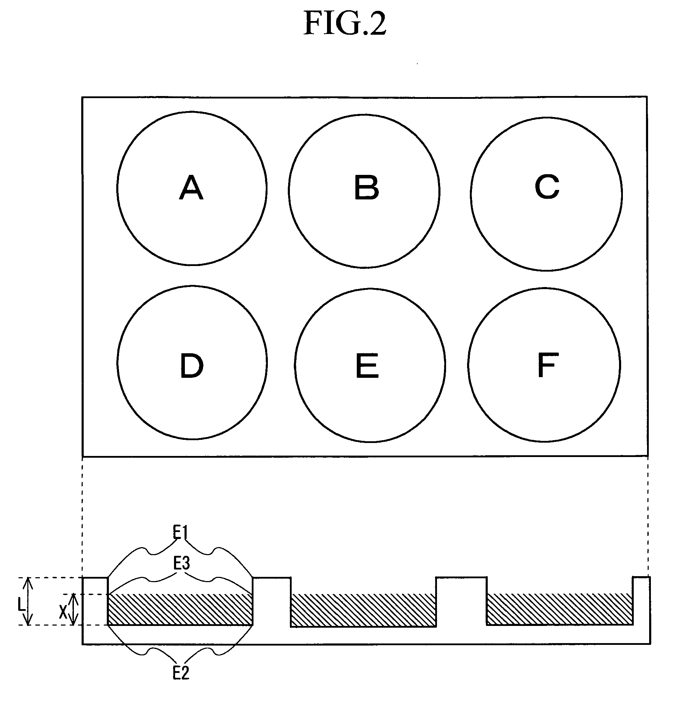

In Fig. 2, an example of the culture vessel 40 which is to be a specimen of the observation object for the microscope 1 in the present embodiment is shown. The culture vessel 40 shown in Fig. 2 has a 6-well plate. Hereinafter, 6 wells in the 6-well plate is called well A, well B, well C, well D, well E, and well F, respectively.

Any type of culture vessel such as a dish or a flask and the like may be adopted as far as it can be transferred onto the stage 4 by a transferring system, which is not shown. Note that, hereinafter, description will be given taking as an example a case of calculation of medium volume of medium in each well using the 6-well plate as the culture vessel 40 shown in Fig. 2.

Conventionally, such a technique has been contemplated to calculate the medium volume based on a color of the medium. However, the color of the medium varies based on not only the variation of medium volume but also the variation of medium pH. Therefore, in the present embodiment, the medium volume is calculated regardless of pH variation and the like.

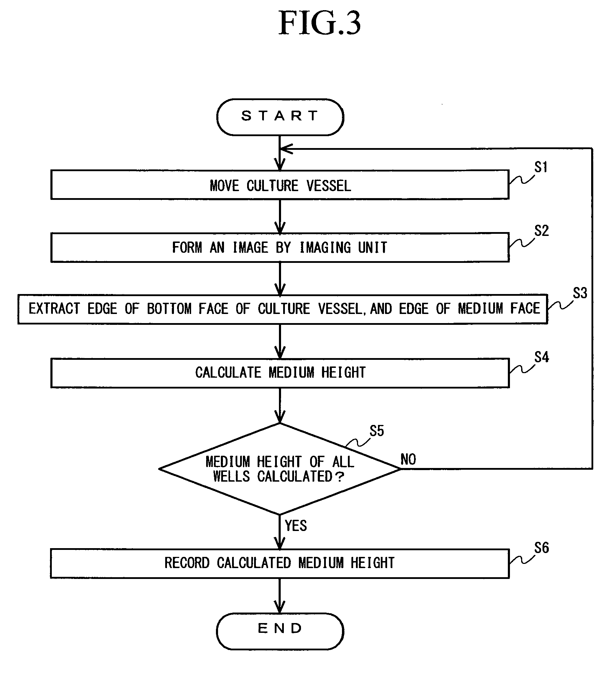

The flow chart of Fig. 3 shows an operation of the computer controlling unit 31 when calculating the medium volume.

In step S1 , the computer controlling unit 31 drives the stage 4 via the microscope controlling unit 9 to move the culture vessel 40.

As described above, the objective lens 5 is provided with the wide-angle lens.
Therefore, distortion tends to occur in the circumference part in the visual field of the objective lens 5. Accordingly, the computer controlling unit 31 moves the culture vessel 40 to a position where the distortion tends to occur caused by the objective lens 5. In the present embodiment, in the first step S1, the stage 4 is moved to such a position that the well A and the well D are disposed in the circumference part in the visual field of the objective lens unit 5 (referred to as a "calculation position" hereinafter).

When the culture vessel 40 is a 35 mm dish, for example, substantially the situation is similar. When the culture vessel 40 is a 75 cm² flask, for example, since the calculation position is the same as the position in a state that the optical axis and the culture vessel are aligned, necessity of movement of the stage 4 as described above does not occur. In other wards, it is enough to move the stage 4 to the optimal calculation position corresponding to the shape of the culture vessel 40.

In step S2, the computer controlling unit 31 drives the imaging unit 6 via the microscope controlling unit 9 to generate an image of the culture vessel 40 by the imaging element 6A.

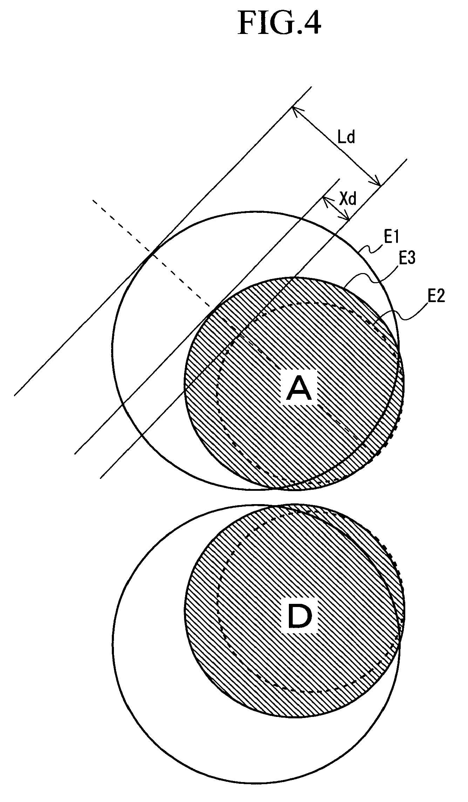

In the present embodiment, as described in step S1, an image of the culture vessel 40 is generated in a position where the distortion tends to occur. Consequently, as shown in Fig. 4, distortion occurs in the generated image. Fig. 4 shows partial images of the well A and the well D, which are shown in Fig. 2, in the culture vessel 40. Note that such distortion is compensated to generate the observation images when observation is carried out.

In step S3, the computer controlling unit 31 obtains an image generated in step S2 via the microscope controlling unit 9, and the bottom face edge of the culture vessel 40 and the medium top face edge is extracted from the image.

In Fig. 4, E1 shows the edge of the top face of the culture vessel 40 (corresponding to E1 of Fig. 2), and E2 shows the bottom face edge of the culture vessel 40 (corresponding to E2 of Fig. 2). In Fig. 4, E3 shows the medium face edge of the culture vessel 40 (corresponding to E3 of Fig. 2). The edge E2 of the bottom face of the culture vessel 40 is always identical regardless of the medium volume. That is, the edge E2 can be determined into a pattern in advance, corresponding to a type (shape) of the culture vessel 40, lens characteristics (such as distortion quantity) of the objective lens unit 5 and imaging condition by the imaging unit 6. The computer controlling unit 31 performs pattern matching using this pattern with the images generated in step S2 to extract the edge E2 of the bottom face of the culture vessel 40.

In addition, the medium is colored usually by pH indexing medicine and the like (oblique lines stand for colored part in Figs. 2 and 4). Then, the computer controlling unit 31 extracts the medium face edge E3 of the culture vessel 40 by extracting the colored part.

The computer controlling unit 31 extracts the edge of each well included in the image obtained in step S2.

It should be noted that such an arrangement may be adopted so that the edge E2 of the bottom face of the culture vessel 40 and the edge E3 of the medium face of the culture vessel 40 are obtained by image analysis.

In step S4, the computer controlling unit 31 calculates the height of the medium based on the edge extracted in step S3.

In Fig. 4, the top face edge E1 of the culture vessel 40 is always identical regardless of the medium volume as the bottom face edge E2 of the culture vessel 40. As shown in Fig. 2, supposing the height from the bottom face to the top face of the culture vessel 40 is L, this height L is constant. Accordingly, the distance Ld shown in Fig. 4 between the top face edge E1 of the culture vessel 40 and the bottom face edge E2 of the culture vessel can be obtained in advance corresponding to the height L, lens characteristics of the objective lens unit 5 and the imaging condition by the imaging unit 6.

Firstly, the computer controlling unit 31 calculates the distance Xd between the bottom edge E2 of the culture vessel 40 and the culture top face edge E3 of the culture vessel 40 which has been extracted in step S3. Then, based on the distance Xd and aforementioned distance Ld, the height X from the bottom of the culture vessel 40 to the face of the medium (refer to Fig. 2) is calculated.

The computer controlling unit 31 calculates the medium height X for each well which is included in the image obtained in step S2.

In step S5, the computer controlling unit 31 determines whether the medium height of all the wells included in the culture vessel 40 have been calculated or not. If it is determined that the medium height of all the wells have been calculated, the computer controlling unit 31 goes to step S6. In the contrary, if it is determined that there are wells the height of which have not been calculated yet, the computer controlling unit 31 returns to step S1 and drives the stage 4 via the microscope controlling unit 9 to move the culture vessel 40 for calculation of the medium height of the next well. That is, in the step S1 of the second time, the well B and the well E shown in Fig. 2 are disposed at the calculation position, and in the step S1 of the third time, the well C and the well F shown in Fig. 2 are disposed at the calculation position.

In step S6, the computer controlling unit 31 stores the medium height of each well calculated in step S4 in a memory, which is not shown, and completes a series of process.

It should be noted that quantity of distortion caused by the objective lens, which is necessary to perform aforementioned series of process, can be calculated in advance corresponding to at least one of a shape of the culture vessel 40, resolution of the imaging unit 6, and required preciseness of medium volume calculation. For example, if the resolution of the imaging unit 6 is high, the necessary distortion quantity becomes small, reversely if the resolution of the imaging unit 6 is low, the necessary distortion quantity becomes large. In addition, if the required preciseness is high, the necessary distortion becomes large, reversely if the required preciseness is low, the necessary distortion becomes small.

In addition, in order to adjust the distortion quantity, such an optical unit may be provided that can be put into and taken out from a light path of the objective lens unit 5 and enhances distortion. When the objective lens unit 5 does not have distortion, an optical unit which generates distortion may be arranged in the light path of the objective lens unit 5 so that the optical unit can be put into and taken out from the light path.

In the flow chart of Fig. 3, such an example was shown that the medium height was calculated (steps S3, S4) whenever the culture vessel 40 was moved (step S1) to generate an image (step S2), however, such arrangement may be adopted that the medium height calculation is performed after the images of all wells are generated. Moreover, such arrangement may be adopted as well that a part of or all of the process are performed in the microscope body 2.

In the flow chart of Fig. 3, such an example was shown that images of the culture vessel were generated two wells by two wells in step S2. However, the following constitution may be acceptable as well, that is, image is generated one well by one well, image is generated three wells by three wells (one image for well A to well C, and one image for well D to well F), and an image is generated including all of six wells. Moreover, such constitution may be accepted that a plurality of images are generated having different number of wells included in one image, and through averaging the medium heights which are calculated based on respective image, the medium height is obtained.

In the flow chart of Fig. 3, such an example was shown that the medium height was calculated based on one image (image that was generated in step S2). However, such arrangement may be adopted that a plurality of images having different focal distances are generated through controlling the stage 4 and the focus controlling unit 8, and the medium height is calculated based on a plurality of images. For example, such arrangement may be adopted that the medium height is calculated based on an image generated through focus adjustment for the bottom face of the culture vessel 40, and an image generated through focus adjustment for a plane near to the intermediate plane between the bottom and the top face of the culture vessel 40.

Furthermore, such arrangement may be adopted as well that a medium height calculation method based on one image described in the flow chart of Fig. 3 and a medium height calculation method based on a plurality of images are used in a selective way, or used in a combination manner thereof.

In the present embodiment, intentional distortion provided at the visual circumference portion of the objective lens unit 5 makes it easy to detect each edge, such as the top face of the well, the edge of the medium liquid level and the edge of the bottom face of the well provided in the culture vessel 40. However, it is possible to detect each edge, such as the edge of the top face of the well, the edge of the medium liquid level and the edge of the bottom face of the well provided in the culture vessel 40, although the distortion is not provided intentionally.

When a wide-angle lens with low magnification is in use as the objective lens unit 5, for example, the culture vessel 40 is in a bird's-eye view in the circumference part in the visual field of the objective lens unit 5. Thereby, even though distortion is not provided intentionally, the top face, the medium liquid level and the bottom face of each well in the culture vessel 40 are imaged with parallax by the imaging unit 6. Performing image processing for these images makes it possible to detect each edge, which are the top face, the medium liquid level and the bottom face of well provided in the culture vessel 40. Hereinafter, calculation of medium volume without intentional distortion will be described. Since process flow of this case is the same as the present embodiment, description uses aforementioned flow chart of Fig. 3. At the same time, steps S2 to S6 in aforementioned flow chart of Fig. 3 will be described in detail.

Step S2: The culture vessel 40 (for example, a vessel in which one or a plurality of wells are formed) are imaged by the imaging unit 6 (a camera having two-dimensional imaging element alley, like a CCD camera, for example) to obtain an image of the culture vessel 40.

Step S3: The computer controlling unit 31 performs processing operation to extract the edge E1 of the top face of the well, the edge E2 of the medium liquid level and the edge E3 of the bottom face of the well, using the images obtained in step S2. This processing operation includes the first image processing operation, in which edge enhancing process is performed for images of each well from the well A to the well F of the culture vessel 40 respectively based on the image of the culture vessel 40 generated by the imaging unit 6, then the edge E1 in the top face of the well, the edge E2 of the medium liquid level and the edge E3 of the bottom face of the well are distinguished using the images to which the edge enhancing process has been performed, to obtain these edges information.

It should be noted that it is not necessary to obtain all of the edge E1 to the edge E3, but it is enough to obtain only either of the two determined edge information in the first image processing operation: specifically, edge information of the edge E2 of the medium liquid level and the edge E1 of the top face of the well; or edge information of the edge E2 of the medium liquid level and the edge E3 of the bottom face of the well.

In the first image processing operation, a macro image that captures the whole image of the culture vessel 40 which the imaging unit 6 has obtained is processed by image segmentation so as to correspond to each well. For that purpose, the macro image of the culture vessel 40 is segmented after the known edge enhancement process has been done, so that overlapping area of three pieces of edge information of the edge E1 of the top face of well, the edge E2 of the medium liquid level and the edge E3 of the bottom face of well is formed to be one section. However, three pieces of edge information can not be always obtained in such wells disposed in the surrounding area of the optical axis center of the objective lens unit 5, in which case one or two pieces of edge information is deemed as one section.

In the first image processing operation, edge information is recorded for each section or for each well. When there are three edge information groups or determined two edge information groups, each edge information is divided into three edge information groups, that is, edge E1 of the top face of well, edge E3 of the medium liquid level, and edge E2 of the bottom face of well, and each well information is recorded.

Step 4: The computer controlling unit 31 performs calculation step of information related to the medium volume such as the medium height and so on, based on the edge information extracted in step S3. This process of the step S4 includes the second image processing operation which calculates: liquid level height information (Xd), which is the distance between both edges viewed in the radial direction from the optical axis of the objective lens unit 5, based on the edge E3 of the medium liquid level and the edge E2 of the bottom face of the well obtained in the first image processing operation; well depth information (Ld), which is the distance between both edges viewed in the radial direction from the optical axis of the objective lens unit 5, based on the edge E2 of the bottom face of the well and the edge E1 of the top face of the well obtained in the first image processing operation. This second image processing operation can provide comparison to the reference well depth information from the determined two edge information (the edge E3 of the medium liquid level and the edge E1 of the top face of the well, or the edge E3 of the medium liquid level and the edge E2 of the bottom face of the well), or respective depth information geometrically.

When the computer controlling unit 31 determines that the liquid level height information (Xd) has been obtained as information of two or more than two image elements of the imaging unit 6 (when calculation standard of the liquid level height information was cleared), the computer unit 31 calculates information related to the medium volume, based on the well height information (Ld) and the liquid level height information (Xd) of the culture vessel 40. Information related to the medium volume is, for example, the medium volume itself or the thickness of the medium, and the like.

When the liquid level height information (Xd) is not information of two or more than two image elements of the imaging unit 6, the computer controlling unit 31 does not calculates the liquid level height information because the liquid level height information is hidden in one image element, thereby detection accuracy declines.

Consequently, the image of each well in the culture vessel 40 obtained by the imaging unit is sorted by the computer controlling unit 31 into a group conforming to the calculation standard of the above liquid face height information and a group not conforming thereto.

Steps S5 and S6: Since the well images not conforming to the calculation standard of the liquid level height information are the images in the surrounding area of the optical axis center of the objective lens unit 5, the computer controlling unit 31 drives the stage 4 to change the relative position of the objective lens unit 5 so that the area of the culture vessel 40 not conforming to the calculation standard of the liquid level height information is moved to the circumference area of the visual field of the objective lens unit 5 to obtain liquid level height information of all wells of the culture vessel 40 and the like.

Note that each of the above mentioned embodiments employs such configuration as to drive the stage 4, however, it is not necessary to limit to this but such configuration may be adopted that the objective lens unit 5 is moved to change the relative position between the stage 4 and the objective lens unit 5 so that imaging area of the culture vessel 40 is changed.

Hereinafter, detecting process of the medium replacement time of the culture vessel 40, and health condition and culture condition of the medium or cell within the culture vessel 40 will be described.

As described above, since the computer controlling unit 31 can measure the medium volume directly, comparison between the measured medium volume and the predetermined threshold makes it possible to detect the culture replacement time of the culture vessel 40. At the same time, as mentioned above, the computer controlling unit 31 performs image analysis process for each well of the culture vessel 40. Especially, the image processing of the image in the overlapped area of the edge E2 of the well bottom face and the edge E1 of the well top face can make it possible to detect health condition, culture condition and the like of the medium or the cell. Moreover, health condition and culture condition of the medium or the cell can be grasped exactly through overall determining and detecting of the measured medium volume, condition of cell division, the number of cores, breeding area of cells, and color change of cells and the like.

As described so far, according to the present invention, the culture vessel 40 is disposed at a position where distortion caused by observation optical system occurs and the culture vessel 40 is imaged so that an image is formed. Then, medium volume of the medium in the culture vessel 40 is calculated based on the generated image. Therefore, using distortion caused by observation optical system, the medium volume in the culture vessel 40 can be calculated without adding any new arrangement. Moreover, detailed culturing condition can be grasped based on the calculated medium volume.

Especially, according to the present embodiment, it is not necessary to add any new arrangement such as the second imaging unit and the like for grasping the medium volume. At the same time, through consideration of the medium volume described in the present embodiment and the medium volume calculated based on the color of the medium, detailed culturing condition including the cause of medium volume change and the like can be grasped.

Technique described in the present embodiment is suitable for an automatic observation device provided with automatic medium replacement arrangement and automatic adding device. For example, it is possible to determine whether consecutive culturing is right or wrong based on the calculated medium volume, to grasp an abnormal situation in culturing, and to determine whether the medium replacement is carried out adequately or not.

In the present embodiment, description was made taking the microscope shown in Fig. 1 as an example of the observation device of the present invention. However, the present invention can be applied to other microscope and observation device in similar way. For example, the present invention is applicable similarly to a microscope provided with both of the macro-optical system and the micro-optical system.

## Claims

1. An observation device which is provided with an observation optical system and observes a specimen via the observation optical system, the specimen being cultured in a culture vessel, comprising:
a moving unit moving the culture vessel to a position where a distortion caused by the observation optical system occurs;
an imaging unit imaging the culture vessel to generate an image at the position where the distortion occurs; and
a calculating unit calculating a medium volume of a medium in the culture vessel based on the image.

2. The observation device according to claim 1, wherein
the calculating unit extracts an edge of a bottom face of the culture vessel and an edge of a face of the medium, and calculates the medium volume based on the edge of the bottom face of the culture vessel and the edge of the face of the medium which are extracted.

3. The observation device according to claim 1, further comprising
an optical unit which can be put into and taken out from a light path of the observation optical system and enhances the distortion.

4. An observation device having an observation optical system and a stage on which a culture vessel being formed with one or a plurality of wells is placed, and observing, via the observation optical system, cells in a medium which is filled in each of the wells, the observation device comprising:
an imaging unit having a plurality of imaging elements and imaging the culture vessel via the observation optical system to generate an image;
an image processing unit processing the image for each of the wells of the culture vessel based on the image generated by the imaging unit to obtain either an edge of a medium liquid level and an edge of a top face of the well or the edge of the medium liquid level and an edge of a bottom face of the well; and
a calculating unit calculating information related to a medium volume of the well based on the edge of the medium liquid level and one of the edge of the top face of the well and the edge of the bottom face of the well which are obtained by the image processing unit.

5. The observation device according to claim 4, wherein
the image processing unit comprises:
a first image processing unit performing edge enhancement process on the image for each of the wells of the culture vessel based on the image generated by the imaging unit and distinguishing to obtain the edge of the top face of the well, the edge of the medium liquid level, and the edge of the bottom face of the well; and
a second image processing unit calculating liquid level height information which is a distance between the edge of the medium liquid level and the edge of the bottom face of the well viewed in radial direction from an optical axis of the observation optical system based on the edge of the medium liquid level and the edge of the bottom face of the well which are obtained by the first image processing unit, and calculating well depth information which is a distance between the edge of the bottom face of the well and the edge of the top face of the well viewed in radial direction from the optical axis of the observation optical system based on the edge of the bottom face of the well and the edge of the top face of the well which are obtained by the first image processing unit, and wherein
the calculating unit calculates the information related to the medium volume based on the well depth information and the liquid level height information when the image processing unit determines that the liquid level height information has been obtained as information of two or more image elements of the imaging unit.

6. The observation device according to claim 5, further comprising:
a condition detecting unit detecting health condition of both the medium and the cells in the well based on an image of an area where an image at inner side of the edge of the top face of the well and an image at inner side of the edge of the bottom face of the well overlap, the edges being obtained by the first image processing unit.

7. The observation device according to claim 4 or 5, wherein
the stage and the observation optical system are moved relatively for the calculating unit to calculate the medium volume of all of the wells of the culture vessel, and the imaging unit images the culture vessel.
